(19) Europäisches Patentamt
European Patent Office
Office européen des brevets

(11) Veröffentlichungsnummer: **0 000 156**
**B1**

(12) **EUROPÄISCHE PATENTSCHRIFT**

(51) Int. Cl.³: **C 07 C 149/42,**
C 07 C 93/14,
C 07 C 147/12,
C 07 D 319/08,
A 01 N 41/02, A 01 N 41/12,
A 01 N 33/18, //
C 07 C 87/60, C 07 C 121/78

(21) Anmeldenummer: **78100172.2**

(22) Anmeldetag: **15.06.78**

(54) **2-Arylamino-3,5-dinitro-benzotrifluoride, deren Herstellung, und deren Verwendung als Schädlingsbekämpfungsmittel**

(30) Priorität: **24.06.77 DE 2728536**

(43) Veröffentlichungstag der Anmeldung:
**10.01.79 Patentblatt 79/01**

(45) Bekanntmachung des Hinweises auf die Patenterteilung:
**12.11.80 Patentblatt 80/23**

(84) Benannte Vertragsstaaten:
**BE CH DE FR GB NL SE**

(56) Entgegenhaltungen:
**DE - A - 2 509 416**

(73) Patentinhaber: **BAYER AG
ZENTRALBEREICH PATENTE,
MARKEN UND LIZENZEN
D - 5090 LEVERKUSEN 1, BAYERWERKE (DE)**

(72) Erfinder: **Hartmann, Alfons, Dr.
Poststrasse 35
D - 6645 Beckingen (DE)
Klauke, Erich, Dr.
Eichendorffweg 8
D - 5068 Odenthal-Hahnenberg (DE)
Hammann, Ingeborg, Dr.
Belfortstrasse 9
D - 5000 Köln (DE)
Roessler, Peter, Dr.
Elsterstrasse 15
D - 5060 Bergisch-Gladbach (DE)
Paul, Volker, Dr.
Ahornstrasse 5
D - 5650 Solingen (DE)**

**EP 0 000 156 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

2-Arylamino-3,5-dinitro benzotrifluoride, deren Herstellung und deren Verwendung als Schädlingsbekämpfungsmittel

Die vorliegende Erfindung betrifft neue 2-Arylamino-3,5-dinitro-benzotrifluoride, Verfahren zu ihrer Herstellung sowie ihre Verwendung als Schädlingsbekämpfungsmittel.

Es sind bereits 2-Arylamino-3,5-dinitro-benzotrifluoride mit insektiziden, akariziden, fungiziden und bakteriziden Eigenschaften bekannt geworden (vgl. Deutsche Offenlegungsschrift 2 509 416). Ihre Wirkung ist jedoch, vor allem bei niedrigen Aufwandkonzentrationen, nicht voll befriedigend.

Zum Stand der Technik wird weiters auf US—PS 2 212 825 verwiesen, aus der folgende Verbindung bekannt ist:

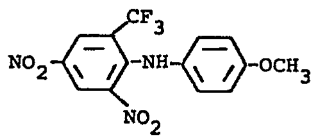

Est ist jedoch nichts über die insektizide Wirksamkeit dieser Verbindung bekannt. Die in US—PS 2 212 825 genannten Verbindungen sind Farbstoffzwischenprodukte.

Es wurden die neuen 2-Arylamino-3,5-dinitro-benzotrifluoride der allgemeinen Formel (1)

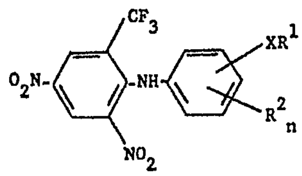

(I)

in welcher

X für O, S, SO oder $SO_2$ steht,

n für eine ganze Zahl von 1 bis 4 steht,

$R^1$ für durch Halogen und/oder Halogenalkoxy substituiertes Alkyl oder für gegebenenfalls durch Halogen, Halogenalkyl, Halogenalkoxy, Halogenalkylmercapto, Halogenalkylsulfonyl substituiertes Phenyl steht und

$R^2$ für gleiche oder verschiedene Reste aus der Gruppe Wasserstoff, Halogen, Cyan, Nitro, gegebenenfalls durch Halogen und/oder Halogenalkoxy substituiertes Alkyl steht.

Wobei ortho-ständige Substituenten $XR^1$ und $R^2$ gemeinsam mit den beiden angrenzenden C-Atomen des Phenylkerns einen gegebenenfalls halogensubstituierten Dioxanylring bilden können; steht X für S, SO oder $SO_2$, kann $R^1$ zusätzlich zu den oben angegebenen Resten für unsubstituiertes Alkyl stehen, gefunden.

Weiterhin wurde gefunden, daß man die neuen 2-Arylamino-3,5-dinitro-benzotrifluoride der Formel (I) erhält, wenn man

a) ein Anilin der allgemeinen Formel (II)

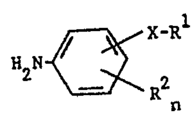

(II)

in welcher

X, $R^1$, $R^2$ und n die oben angegebene Bedeutung haben, mit 2-Chlor-3,5,-dinitro-benzotrifluorid der Formel (III)

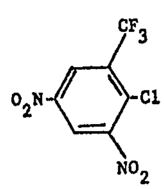

(III)

in Gegenwart eines säurebindenden Mittels sowie gegebenenfalls in Gegenwart eines Verdünnungsmittels umsetzt, oder

b) 2-Amino-3,5-dinitro-benzotrifluorid der Formel (IV)

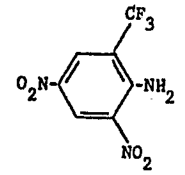

(IV)

mit einer Verbindung der allgemeinen Formel (V)

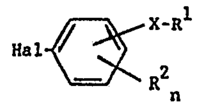

(V)

in welcher
X, $R^1$, $R^2$ und n die oben angegebene Bedeutung haben und Hal für Fluor, Chlor oder Brom steht, in Gegenwart eines säurebindenden Mittels, sowie gegebenenfalls in Gegenwart eines Verdünnungsmittels umsetzt, oder
c) eine Verbindung der allgemeinen Formel (I) die nach Methode a) oder b) hergestellt wurde und in der $R^1$, $R^2$ und n die oben angegebene Bedeutung haben und X für S steht, mit Hilfe von Oxidationsmitteln, gegebenenfalls in Gegenwart eines Verdünnungsmittels, in entsprechende Verbindungen der Formel (I) überführt, in welchen X für SO oder $SO_2$ steht.

Überraschenderweise zeigen die neuen 2-Arylamino-3,5-dinitro-benzotrifluoride der Formel (I) eine höhere insektizide, akarizide, entwicklungshemmende, fungizide und bakterizide Potenz als die aus der Deutschen Offenlegungsschrift 2 509 416 bekannten chemisch naheliegenden Verbindungen gleicher Wirkungsrichtung.

Die erfindungsgemäßen Stoffe stellen somit eine Bereicherung der Technik dar.

Verwendet man 2-Chlor-3,5-dinitro-benzotrifluorid und 4-Tri-fluormethoxy-anilin als Ausgangsstoffe, so läßt sich der Reaktionsablauf durch das folgende Formelschema wiedergeben:

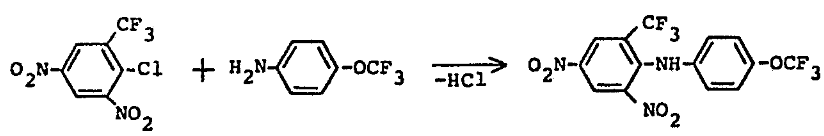


Die als Ausgangsstoffe zu verwendenden Verbindungen II, III, IV und V sind bekannt oder lassen sich nach bekannten Verfahren herstellen.

Vorzugsweise werden die neuen Wirkstoffe durch Umsetzung von 2-Chlor-3,5-dinitro-benzotrifluorid (III) mit einem Anilin der Formel (II) hergestellt, in welcher X für O, S oder $SO_2$, $R^1$ für Alkyl mit 1 bis 4 C-Atomen, Halogenalkyl mit 1 bis 4 C-Atomen oder gegebenenfalls durch Chlor oder Trifluormethylsulfonyl substituiertes Phenyl, $R^2$ für Wasserstoff, Halogen oder Trifluormethyl und n für 1 oder 2 steht, oder in welcher X—$R^1$ und $R^2$ einen durch Fluor substituierten, an den Phenylkern ankondensierten 1,3-Dioxan-Ring darstellen. Als Beispiele für erfindungsgemäß verwendbare Aniline der Formel (II) seien genannt:

2-Trifluormethoxy-anilin
3-Trifluormethoxy-anilin
4-Trifluormethoxy-anilin
2-Chlor-4-trifluormethoxy-anilin
3-Chlor-4-trifluormethoxy-anilin
2-Trifluormethylmercapto-anilin
3-Trifluormethylmercapto-anilin
4-Trifluormethylmercapto-anilin
2-Chlor-5-trifluormethylmercapto-anilin
3-Chlor-4-trifluormethylmercapto-anilin
3-Chlor-4-chlordifluormethylmercapto-anilin
4-Methylsulfonyl-anilin
4-Trifluormethylsulfonyl-anilin
2-Chlor-4-trifluormethylsulfonyl-anilin
2-Chlor-5-trifluormethylsulfonyl-anilin
2,6-Dichlor-4-trifluormethylsulfonyl-anilin
6-Amino-2,2,4,4-tetrafluor-1,3-benzodioxan
2-Amino-5-methylmercapto-benzotrifluorid
3-Amino-4-methylmercapto-benzotrifluorid

2-Amino-5-methylsulfonyl-benzotrifluorid
3-Amino-4-ethylsulfonyl-benzotrifluorid
5-Amino-2(4-chlorphenoxy)-benzotrifluorid
4-(4-Trifluormethylsulfonyl-phenoxy)-anilin
2-Amino-5-(4-chlorphenylthio)-benzotrifluorid
3-Amino-4-(4-chlorphenylthio)-benzotrifluorid
2-Amino-diphenylsulfon

Besonders bevorzugt sind Verbindungen der allgemeinen Formel (I), in welcher X für O, S oder $SO_2$, $R^1$ für Trifluormethyl oder gegebenenfalls durch Chlor oder Trifluormethylsulfonyl substituiertes Phenyl, $R^2$ für Wasserstoff, Chlor oder Trifluormethyl und n für 1 steht, oder in welcher X—$R^1$ und $R^2$ einen durch Fluor substituierten, an den Phenylkern ankondensierten 1,3-Dioxan-Ring darstellen. $R^1$ steht außerdem bevorzugt für Methyl oder Ethyl, wenn X für S oder $SO_2$ steht.

Als Verdünnungsmittel für die Verfahrensvarianten a) und b) eignen sich inerte organische Lösungsmittel. Bevorzugt werden Dimethylformamid oder Tetrahydrofuran verwendet. Zuweilen ist es auch vorteilhaft, in wäßriger Suspension zu arbeiten.

Als säurebindende Mittel eignen sich Basen wie Alkali- oder Erdalkalimetallhydroxide, -carbonate oder -hydride. Bevorzugt verwendet man Kaliumhydroxid, Natriumhydrid oder Natriumhydrogencarbonat.

Die Reaktionstemperaturen können in einem größeren Bereich variiert werden. Im allgemeinen arbeitet man zwischen −10 und +150°, vorzugsweise bei 10 bis 40°, wenn ein organisches Lösungsmittel benutzt wird, und bei 100°, wenn in wäßriger Suspension gearbeitet wird. Im letzteren Falle kann die Temperatur durch Verwendung eines Druckgefäßes noch gesteigert werden.

Gewöhnlich werden die Reaktionspartner in äquimolaren Mengen eingesetzt, doch auch die Verwendung der einen oder anderen Komponente im Überschuß ist möglich.

Zur Durchführung der Darstellungsmethode c) arbeitet man vorzugsweise in Eisessig als Lösungsmittel und benutzt Wasserstoffperoxid als Oxidationsmittel. Zur Darstellung der Sulfoxide setzt man mit äquimolaren Mengen vorzugsweise bei 10—30°, zur Darstellung der Sulfone mit mindestens der doppelt molaren Menge Wasserstoffperoxid vorzugsweise bei der Siedetemperatur des Lösungsmittels um.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea madarae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pamphigus spp., Pediculus humanus corporis, Haematopinus spp., Linognathus spp.

Aus der Ordnung der Mallophage z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp. Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius

obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die erfindungsgemäßen Wirkstoffe weisen eine starke fungitoxische und bakteriotoxische Wirkung auf. Sie schädigen Kulturpflanzen in den zur Bekämpfung von Pilzen und Bakterien notwendigen Konzentrationen nicht. Aus diesen Gründen sind sie für den Gebrauch als Pflanzenschutzmittel zur Bekämpfung von Pilzen und Bakterien geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemäßen Wirkstoffe haben ein breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger.

Die erfindungsgemäßen Verbindungen wirken gegen Pilze und Bakterien, die verschiedene Kulturpflanzen befallen, wie z.B. Pythium-Arten, Phytophthora-Arten, Fusarium- Arten, Verticillium alboatrum, Phialophora cinerescens, Sclerotinia sclerotiorum, Botrytis-Arten, Cochliobolus miyabeanus, Mycosphaerella musicola, Cercospora personata, Helminthosporium gramineum, Alternaria-Arten, Colletotrichum-Arten, Venturia inaequalis, Rhizoctonia-Arten, Thielaviopsis basicola Xanthomonas oryzae und Pseudomonas Lachrymans. Die erfindungsgemäßen Verbindungen wirken sowohl gegen Getreiderkrankheiten, wie z.B. Puccinia recondita, Erysiphe graminis, Tilletia caries als auch gegen Reiskrankheiten, wie z.B. Pyriculania oryzae, Pellicularia sasakii.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwending von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gestine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und

Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Bei der Verwendung als Blattfungizide können die Wirkstoffkonzentrationen in den Anwendungsformen in einem größeren Bereich variiert werden. Sie liegen im allgemeinen zwischen 0,1 und 0,00001 Gewichtsprozenten, vorzugsweise zwischen 0,05 und 0,0001 %.

Bie der Saatgutbehandlung werden im allgemeinen Wirkstoffmengen von 0,001 bis 50 g je Kilogramm Saatgut, vorzugsweise 0,01 bis 10 g benötigt.

Zur Bodenbehandlung sind Wirkstoffmengen von 1 bis 1000 g je cbm Boden, vorzugsweise von 10 bis 200 g, erforderlich.

## Beispiel A

Tetranychus-Test (resistent)

| | | |
|---|---|---|
| Lösungsmittel: | 3 Gewichtsteile | Dimethylformamid |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung werden Bohnenpflanzen (Phaseolus vulgaris), die stark von allen Entwicklungsstadien der gemeinen Spinnmilbe oder Bohnenspinnmilbe (Tetranychus urticae) befallen sind, tropfnaß besprüht.

Nach den angegebenen Zeiten wird die Abtötung in % bestimmt. Dabei bedeutet 100%, daß alle Spinnmilben abgetötet wurden; 0% bedeutet, daß keine Spinnmilben abgetötet wurden.

Wirkstoffe, Wirkstoffkonzentrationen, Auswertungszeiten und Resultate gehen aus der nachfolgenden Tabelle hervor:

TABELLE

(pflanzenschädigende Milben)

Tetranychus-Test

| Wirkstoffe | Wirkstoffkonzentration in % | Abtötungsgrad in % nach 2 Tagen |
|---|---|---|
| 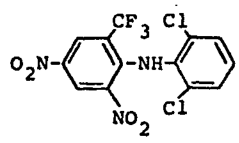 | 0,001 | 98 |
| | 0,0001 | 0 |
| (bekannt) | | |
| 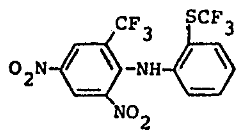 | 0,001 | 100 |
| | 0,0001 | 50 |
| 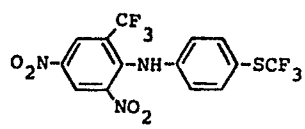 | 0,001 | 100 |
| | 0,0001 | 99 |
| 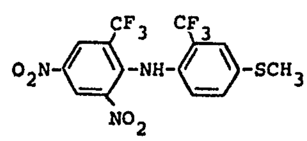 | 0,001 | 98 |
| | 0,0001 | 50 |
| 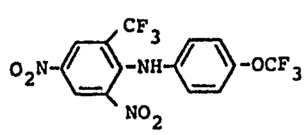 | 0,001 | 100 |
| | 0,0001 | 70 |
| 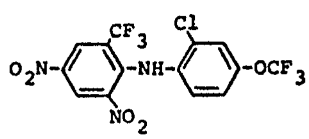 | 0,001 | 100 |
| | 0,0001 | 100 |

TABELLE (Fortsetzung)

(pflanzenschädigende Milben)

Tetranychus-Test

| Wirkstoffe | Wirkstoffkonzentration in % | Abtötungsgrad in % nach 2 Tagen |
|---|---|---|
| 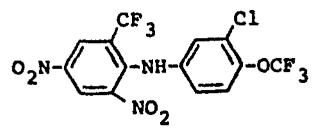 | 0,001<br>0,0001 | 100<br>100 |
| 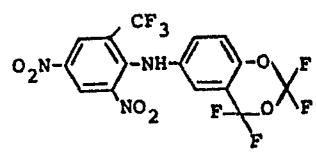 | 0,001<br>0,0001 | 100<br>100 |
| 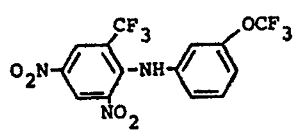 | 0,001<br>0,0001 | 100<br>95 |
| 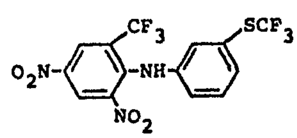 | 0,001<br>0,0001 | 100<br>95 |
| 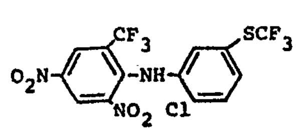 | 0,001<br>0,0001 | 100<br>100 |

## Beispiel B

Phaedon-Larven-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit der Wirkstoffzubereitung besprüht man Kohlblätter (Brassica oleracea) tropfnaß und besetzt sie mit Meerrettichblattkäfer-Larven (Phaedon Cochleariae).

Nach den angegebenen Zeiten wird die Abtötung in % bestimmt. Dabei bedeutet 100%, daß alle Käfer-Larven abgetötet wurden; 0% bedeutet, daß keine Käfer-Larven abgetötet wurden.

Wirkstoffe, Wirkstoffkonzentrationen, Zeiten der Auswertung und Resultate gehen aus der nachfolgenden Tabelle hervor:

TABELLE

(pflanzenschädigende Insekten)

Phaedon Larven-Test

| Wirkstoffe | Wirkstoffkonzentration in % | Abtötungsgrad in % nach 3 Tagen |
|---|---|---|
| 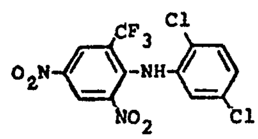 | 0,01 | 100 |
| | 0,001 | 0 |
| (bekannt) | | |
| 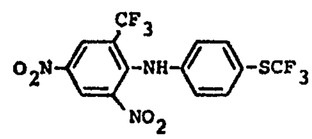 | 0,01 | 100 |
| | 0,001 | 50 |
| 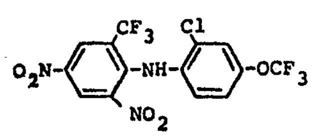 | 0,01 | 100 |
| | 0,001 | 85 |
| 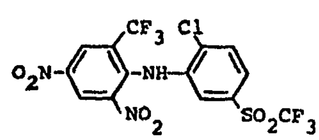 | 0,01 | 100 |
| | 0,001 | 80 |

Durch die im folgenden angegebenen Versuche wird die arthropodenentwicklungshemmende Wirkung der erfindungsgemäßen Verbindungen gezeigt, ohne eine Beschränkung hinsichtlich der Wirkungsbreite dieser Verbindungen vornehmen zu wollen. Dabei werden während der gesamten angegebenen Entwicklung der Testtiere die morphologischen Veränderungen, wie zur Hälfte verpuppte Tiere, unvollständig geschlüpfte Larven oder Raupen, defekte Flügel, pupale Kutikula bei Imagines etc., als Mißbildungen gewertet. Die Summe der morphologischen Mißbildungen, zusammen mit den während des Häutungsgeschehens oder der Metamorphose abgetöteten Tieren, wird in Prozent der Versuchstiere angegeben.

Beispiel C

Entwicklungshemmende Wirkung

Testtier: Laphygma exempta (Eier)
Futter: Mais (Zea mays)
Lösungsmittel: 10 Gew.-Teile Aceton
Emulgator: 1 Gew.-Teil Alkylarylpolyglykoläther

Zur Herstellung einer zweckmäßigen Wirkstoffzubereitung vermischt man 2 Gew.-Teile Wirkstoff mit der angegebenen Menge Lösungsmittel, Emulgator und soviel Wasser, daß eine 1%iger Mischung entsteht, die mit Wasser auf die gewünschte Konzentration verdünnt wird.

Eigelege mit 30 Eiern auf Filterpapier werden mit 1 ml Wirkstofflösung der angegebenen Konzentration in ppm (parts pro million) angefeuchtet und in Kunststoffdosen bis zum Schlupf der Junglarven beobachtet. Die Junglarven werden mit Maisblättern gefüttert, die mit der angegebenen Konzentration der Wirkstofflösung am gleichen Tag wie die Eier besprüht wurden.

Die Entwicklung der Versuchstiere wird bis zur Larve des 3. Stadiums beobachtet.

Zur Kontrolle werden Eigelege in gleicher Weise mit Lösungsmittel und Emulgator der entsprechenden Konzentration behandelt und mit Maisblättern gefüttert. Die Ergebnisse gehen aus nachfolgender Tabelle hervor.

TABELLE

Entwicklungshemmende Wirkung

Laphygma exempta (Eier)

| Wirkstoff | % Entwicklungshemmung bei einer Konzentration von 10 ppm |
|---|---|
| Kontrolle | 0 % |
| 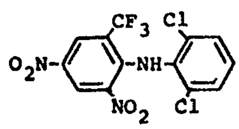 (bekannt) | 20 % |
| 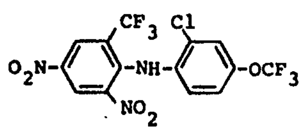 | 100 % |
| 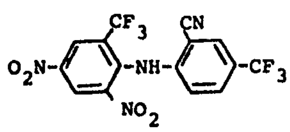 | 100 % |
| 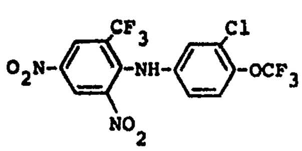 | 100 % |

Beispiel D

Entwicklungshemmende Wirkung

Testtier: Ceratitis capitata (Eier) 20 Stück
Futter: Kunstfutter (Möhrenpulver mit Hefepulver)
Lösungsmittel: 20 Gew.-Teile Aceton
Emulgator: 5 Gew.-Teile Alkylarylpolyglykoläther

Zur Herstellung einer zweckmäßigen Wirkstoffzubereitung vermischt man 4 Gew.-Teile Wirkstoff mit der angegebenen Menge Lösungsmittel, Emulgator und soviel Wasser, daß eine 1 %ige Mischun' entsteht, die mit Wasser auf die gewünschte Konzentration verdünnt wird.

Die Eier der Testtiere werden auf eine Schale mit Kunstfutter gebracht, in das die gewählte Konzentration der Wirkstoffmischung eingearbeitet ist, sodaß die angegebene Wirkstoffmenge in ppm (parts pro million) erreicht wird. Es wird die Entwicklung bis zum Schlupf der Fliegen beobachtet.

Zur Kontrolle wird nur mit Lösungsmittel und Emulator der angegebenen Konzentration vermischtes Kunstfutter angeboten. Die Ergebnisse gehen aus nachfolgender Tabelle hervor.

TABELLE

Entwicklungshemmende Wirkung

Ceratitis capitata

| Wirkstoff | % Entwicklungshemmung bei einer Konzentration von 10 ppm |
|---|---|
| Kontrolle | 0 % |
| 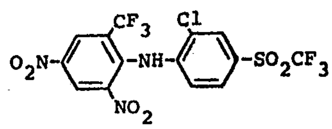 | 100 % |
| 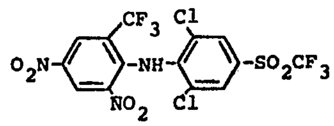 | 100 % |
| 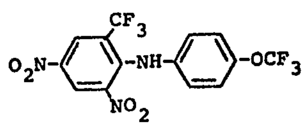 | 100 % |
| 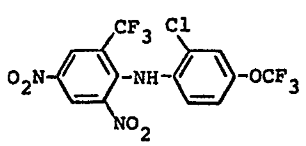 | 100 % |
| 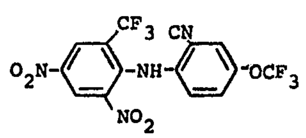 | 100 % |
| 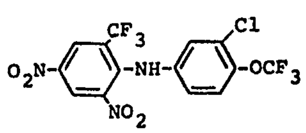 | 100 % |

Beispiel E

Agarplatten-Test

Verwendeter Nährboden:

20 Gewichtsteile Agar-Agar
200 Gewichtsteile Kartoffeldekokt
5 Gewichtsteile Malz
15 Gewichtsteile Dextrose
5 Gewichtsteile Pepton
2 Gewichtsteile $Na_2HPO$
0,3 Gewichtsteile $Ca(NO_3)_2$

Verhältnis von Lösungsmittelgemisch zum Nährboden:

2 Gewichtsteile Lösungsmittelgemisch
100 Gewichtsteile Agarnährboden

Zusammensetzung Lösungsmittelgemisch

0,19 Gewichtsteile DMF oder Aceton
0,01 Gewichtsteile Emulgator Alkylarylpolyglykoläther
*1,80 Gewichtsteile Wasser*
2 Gewichtsteile Lösungsmittelgemisch

Man vermischt die für die gewünschte Workstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlicher vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten und zwei Bakterien beimpft und bei etwa 21°C inkubiert.

Die Auswertung erfolgt je nach der Wachstumsgeschwindigkeit der Organismen nach 4—10 Tagen. Bei der Auswertung wird das radiale Wachstum auf den behandelten Nährböden mit dem Wachstum auf dem Kontrollnährboden verglichen. Die Bonitierung des Erregerwachstums geschieht mit folgenden Kennzahlen:

1 kein Wachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Wirkstoffe, Wirkstoffkonzentrationen und Resultate gehen aus der nachfolgenden Tabelle hervor:

TABELLE

Pilze und Bakterien

| Agarplatten-Test<br>Wirkstoffe | Wirkstoffkonzentration ppm | Fusarium culmorum | Sclerotinia sclerotiorum | Fusarium nivale | Colletotrichum coffeanum | Rhizoctonia solani | Pythium ultimum | Cochliobolus miyabeanus | Botrytis cinerea | Verticillium alboatrum | Pyricularia oryzae | Phialophora cinerescens | Helminthosporium gramineum | Mycosphaerella musicola | Phytophthora cactorum | Venturia inaequalis | Pellicularia sasakii | Xanthomonas oryzae | Pseudomonas Lachrymans |
|---|---|---|---|---|---|---|---|---|---|---|---|---|---|---|---|---|---|---|---|
| 'Zineb'<br>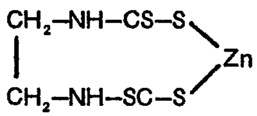<br>bekannt | 10 | 9 | 9 | 9 | 9 | 9 | 5 | 9 | 9 | 9 | 9 | 9 | 5 | 5 | 9 | – | 9 | 9 | – |
| 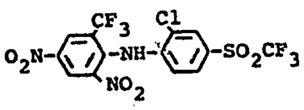 | | 1 | – | 1 | 3 | 1 | 3 | 1 | 1 | 3 | 1 | 5 | 1 | 1 | 1 | – | 1 | 1 | – |
| 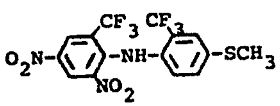 | | 1 | 1 | 1 | 1 | 1 | 3 | 1 | 3 | 1 | 1 | 1 | 1 | 1 | 1 | – | 1 | – | – |
| 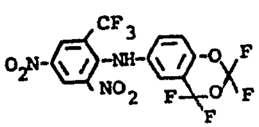 | | 1 | 1 | 1 | 1 | – | 1 | 1 | 1 | 1 | 1 | 1 | 1 | 1 | 1 | – | 1 | 1 | 2 |

# TABELLE

## Pilze und Bakterien

| Agarplatten-Test<br>Wirkstoffe | Wirkstoffkonzentration ppm | Fusarium culmorum | Sclerotinia sclerotiorum | Fusarium nivale | Colletotrichum coffeanum | Rhizoctonia solani | Pythium ultimum | Cochliobolus miyabeanus | Botrytis cinerea | Verticillium alboatrum | Pyricularia oryzae | Phialophora cinerescens | Helminthosporium gramineum | Mycosphaerella musicola | Phytophthora cactorum | Venturia inaequalis | Pellicularia sasakii | Xanthomonas oryzae | Pseudomonas Lachrymans |
|---|---|---|---|---|---|---|---|---|---|---|---|---|---|---|---|---|---|---|---|
| 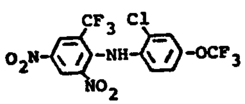 | 10 | 1 | 1 | 1 | 1 | 1 | 1 | 1 | 1 | 1 | 1 | 1 | 1 | 1 | 1 | – | 1 | 1 | – |
| 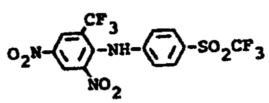 | | 1 | 1 | 5 | 1 | – | – | 5 | – | – | 2 | – | 5 | 3 | 1 | – | 1 | 1 | 1 |
| 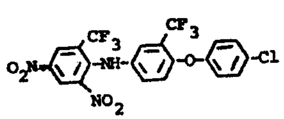 | | 1 | 3 | 3 | 5 | 1 | – | – | – | – | 1 | – | 1 | 5 | 1 | – | 5 | – | – |

Herstellungsbeispiele

1. 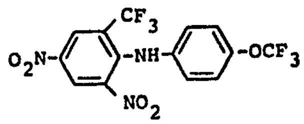

Eine Suspension von 27,0 g (0,1 Mol) 2-Chlor-3,5-dinitro-benzotrifluorid und 17,7 g (0,1 Mol) 4-Trifluormethoxy-anilin in 200 ml Wasser wird zum Sieden erhitzt. Im Verlauf von 30 Min. trägt man 9,3 g (0,11 Mol) Natriumhydrogencarbonat ein und erhitzt weitere 2 Stdn. unter Rückfluß. Man läßt über Nacht bei Raumtemperatur stehen, wobei sich ein gelber, kristalliner Festkörper abscheidet. Nach Absaugen und Waschen mit Petroläther erhält man 36,8 g 2-(4-Trifluormethoxy-anilino)-3,5-dinitro-benzotrifluorid vom Fp. 104°.

Analog Beispiel 1 erhalt man die folgenden Verbindungen:

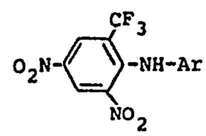

| Beispiel Nr. | Ar | Fp (°C) |
|---|---|---|
| 2 | 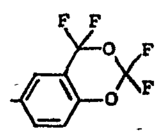 | 125 |
| 3 | 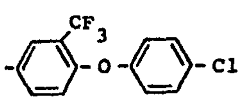 | 174 |
| 4 | 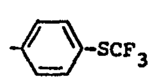 | 108 |
| 5 | 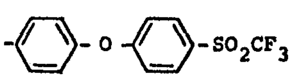 | 138 |

6. 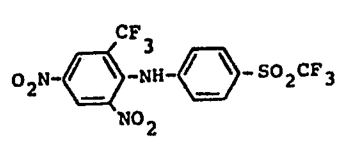

Eine lösung von 11,2 g (50 mMol) 4-Trifluormethylsulfonyl-anilin in 100 ml trockenem Dimethylformamid wird bei Raumtemperatur portionsweise mit 5,6 g (100 mMol) pulvrisierten Kaliumhydroxid versetzt. Anschließend wird bei 25° eine Lösung von 13,6 g (50 mMol) 2-Chlor-3,5-dinitrobenzotrifluorid in 80 ml trockenem Dimethylformamid zugetropft. Man rührt über Nacht bei Raumtemperatur, filtriert, versetzt das Filtrat mit 100 ml Eisessig und trägt auf Eis aus. Man saugt 11,5 g 2-(4-Trifluormethylsulfonyl-anilino)-3,5-dinitro-benzotrifluorid in Form von gelben Kristallen ab. Fp. 172°.

Analog Beispiel 6 wurden die folgenden Verbindungen gewonnen:

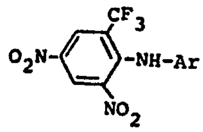

| Beispiel Nr. | Ar | Fp (°C) |
|---|---|---|
| 7 | 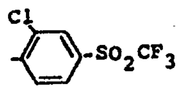 | 157 – 158 |
| 8 | 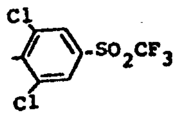 | 125 |
| 9 | 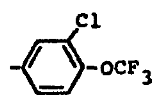 | ($n_D^{20}$ 1,576) |
| 10 | 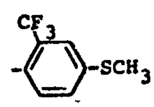 | 115 |
| 11 | 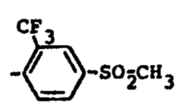 | 192 |
| 12 | 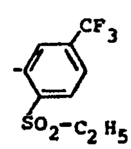 | 176 |

13.

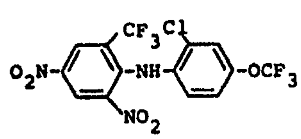

Zu einer Suspension von 5 g Natriumhydrid (mit 20% Paraffin überzogen) in 100 ml trockenem Dimethylformamid gibt man bei 0°C eine Lösung von 21,2g (0,1 Mol) 2-Chlor-4-triluormethoxy-anilin in 200 ml Dimethylformamid. Man rührt 1 Std. bei 25°, kühlt auf 10° ab und tropft eine Lösung von

27,1 g (0,1 Mol) 2-Chlor-3,5-dinitro-benzotrifluorid in 250 ml Dimethylformamid zu. Nach Stehen über Nacht wird filtriet, mit 500 ml Eisessig versetzt und auf ßis gegossen, wobei sich ein dunkles Oel abscheidet. Es wird abgetrennt und im Vakuum von Lösungsmittelresten befreit. So erhielt man 35,4 g 2-(2-Chlor-4-trifluormethoxy-anilino)-3,5-dinitro-benzotrifluorid als viskose Masse, $n^{20}$ -1,574.

Auf ähnliche Weise lassen sich die folgenden Verbindungen darstellen:

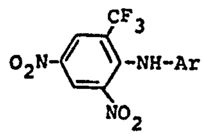

| Beispiel Nr. | Ar | Fp (°C) |
|---|---|---|
| 14 | 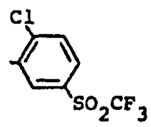 | 110 |
| 15 | 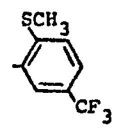 | 140 |
| 16 | 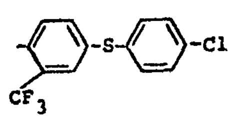 | 104 |
| 17 | 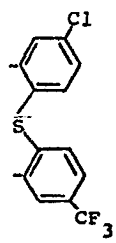 | 124 |
| 18 | 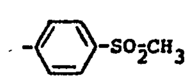 | 172 |
| 19 | 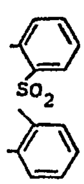 | 114 |
| 20 | 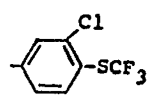 | dunkles, zähes Oel |
| 21 | 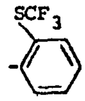 | 95 |

| Beispiel Nr. | Ar | Fp (°C) |
|---|---|---|
| 22 | 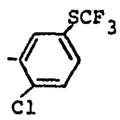 | ($n_D^{20} \approx 1,574$) |
| 23 | 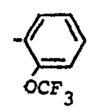 | |
| 24 | 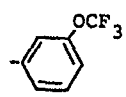 | 114 |
| 25 | 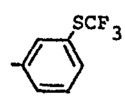 | 78 – 79 |

26. 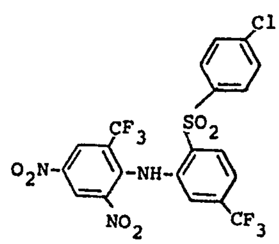

5,4 g (10 mMol) 2-[2-(4-Chlorphenylthio)-5-trifluormethyl]-3,5-dinitro-benzotrifluorid (Herstellungsbeispiel Nr. 17) in 100 ml Eisessig werden mit 3 ml 35 %igem Wasserstoffperoxid versetzt und 3 Min. unter Rückfluß erhitzt. Nach Abkühlen gibt man in 1 ltr. Eiswasser. Die Kristalle von 2-[2-(4-Chlorphenylsulfonyl)-5-trifluormethyl]-3,5-dinitro-benzotrifluorid werden abgesaugt und mit Wasser und Petroläther gewaschen.

Ausbeute 5,6 g, Fp. 191°.

Auf ähnliche Weise erhält man aus der nach Beispiel 15 hergestellten Verbindung:

27. 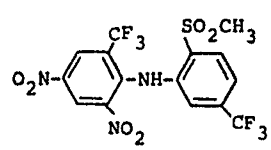 Fp. 185°

## Patentansprüche

1. 2-Arylamino-3,5-dinitrobenzotrifluoride der allgemeinen Formel (I)

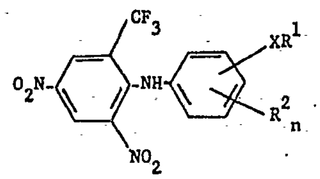

(I)

in welcher
X für O, S, SO oder $SO_2$ steht,
n für eine ganze Zahl von 1 bis 4 steht,
$R^1$ für durch Halogen und/oder Halogenalkoxy substituiertes Alkyl oder für gegebenenfalls durch Halogen, Halogenalkyl, Halogenalkoxy, Halogenalkylmercapto, Halogenalkylsulfonyl substituiertes Phenyl steht und
$R^2$ für gleiche oder verschiedene Reste aus der Gruppe Wasserstoff, Halogen, Cyan, Nitro, gegebenenfalls durch Halogen und/oder Halogenalkoxy substituiertes Alkyl steht, wobei ortho-ständige Substituenten $XR^1$ und $R^2$ gemeinsam mit den beiden angrenzenden C-Atomen des Phenylkerns einen gegebenenfalls halogensubstituierten Dioxanylring bilden können; steht X für S, SO oder $SO_2$, kann $R_1$ zusätzlich zu den oben angegebenen Resten für unsubstituiertes Alkyl stehen.

2. Verfahren zur Herstellung der 2-Arylamino-3,5-dinitro-benzotrifluoride der allgemeinen Formel (I) gemäß Anspruch 1, dadurch gekennzeichnet, daß man.
a) ein Anilin der allgemeinen Formel (II)

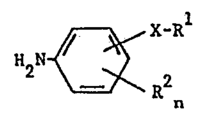

(II)

in welcher
X, $R^1$, $R^2$ und n die oben angegebene Bedeutung haben, mit 2-Chlor-3,5-dinitro-benzotrifluorid der Formel (III)

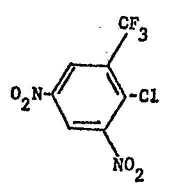

(III)

in Gegenwart eines säurebindenden Mittels sowie gegebenenfalls in Gegenwart eines Verdünnungsmittels umsetzt, oder
b) 2-Amino-3,5-dinitro-benzotrifluorid der Formel (IV)

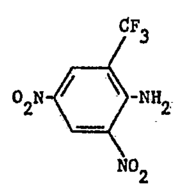

(IV)

mit einer Verbindung der allgemeinen Formel (V)

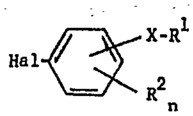

(V)

in welcher
X, $R^1$, $R^2$ und n die oben angegebene Bedeutung haben und Hal für Chlor oder Brom steht, in Gegenwart eines säurebindenden Mittels, sowie gegebenenfalls in Gegenwart eines Verdünnungsmittels umsetzt, oder
c) eine Verbindung der allgemeinen Formel (I), die nach Methode a) oder b) hergestellt wurde und

in der $R^1$, $R^2$ und n die oben angegebene Bedeutung haben und X für S steht, mit Hilfe von Oxidationsmitteln, gegebenenfalls in Gegenwart eines Verdünnungsmittels, in entsprechende Verbindungen der Formel (I) überführt, in welchen X für SO oder $SO_2$ steht.

3. Schädlingsbekämpfungsmittel, gekennzeichnet durch einen Gehalt an 2-Arylamino-3,5-dinitrobenzotrifluoride der allgemeinen Formel (I) gemäß Anspruch 1.

4. Verwendung von 2-Arylamino-3,5-dinitrobenzotrifluoride der allgemeinen Formel (I) gemäß Anspruch 1 zur Schädlingsbekämpfung.

5. Verfahren zur Herstellung von Schädlingsbekämpfungsmitteln, dadurch gekennzeichnet, daß man 2-Arylamino-3,5-dinitrobenzotrifluoride der allgemeinen Formel (I) gemäß Anspruch 1 mit Streckmitteln und/oder oberflächenaktiven Stoffen vermischt.

**Revendications**

1. 2-Arylamino-3,5-dinitrophénylfluoroformes de formule générale

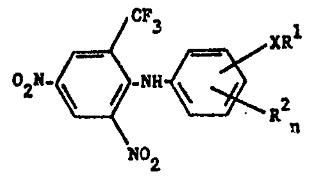

(I)

dans laquelle:

X représente O, S, SO ou $SO_2$,

n représente un nombre entier de 1 à 4,

$R^1$ représente un groupe alkyle substitué par un halogène et/ou un groupe halogénoalcoxy, ou un groupe phényle éventuellement substitué par un halogène ou un groupe halogénoalkyle, halogénoalcoxy, halogénoalkylmercapto, halogénoalkysulfonyle, et le restes $R^2$ sont identiques ou différents et choisis parmi l'hydrogène, les halogènes, les groupes cyano, nitro et alkyle éventuellement substitué par un halogène et/ou un groupe halogénoalcoxy, les substituants $XR^1$ et $R^2$ en ortho pouvant former ensemble avec les deux atomes de carbone du noyau phényle un noyau dioxannyle éventuellement halogéné; si X représente S, SO ou $SO_2$, le reste $R^1$ peut en outre représenter un groupe alkyle non substitué.

2. Procédé pour la préparation de 2-arylamino-3,5-dinitro-phénylfluoroformes de formule générale (I) selon la revendication 1, caractérisé en ce que:

a) on fait réagir une aniline de formule générale (II)

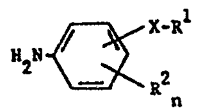

(II)

dans laquelle:

X, $R^1$, $R^2$ et n ont la signification indiquée ci-dessus, avec le 2-chloro-3,5-dinitrophénylfluoroforme de formule (III)

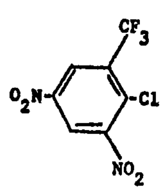

(III)

en présence d'un accepteur d'acide, ainsi éventuellement que d'un agent diluant, ou bien

b) on fait réagir un 2-amino-3,5-dinitrophénylfluoroforme de formule (IV)

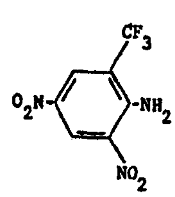

(IV)

avec un composé de formule générale (V)

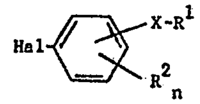

(V)

dans laquelle:

X $R^1$, $R^2$ et n ont la signification indiquée ci-dessus et Hal représente le chlore ou le brome, en présence d'un accepteur d'acide, ainsi éventuellement que d'un agent diluant, ou bien

c) on transforme un composé de formule générale (I), qui a été préparé par la méthode a) ou b) et dans lequel $R^1$, $R^2$ et n ont la signification indiquée ci-dessus et X représente le soufre, en composé correspondant de formule (I) dans laquelle X représente SO ou $SO_2$, au moyens d'agents oxydants, éventuellement en présence d'un agent diluant.

3. Agents de lutte contre les parasites, caractérisés en ce qu'ils contiennent des 2-arylamino-3,5-dinitrophénylfluoroformes de formule générale (I) selon la revendication 1.

4. Utilisation des 2-arylamino-3,5-dinitrophénylfluoroformes de formule générale (I) selon la revendication 1 pour la lutte contre les parasites.

5. Procédé pour la préparation d'agents de lutte contre les parasites, caractérisé en ce que l'on mélange des 2-arylamino-3,5-dinitrophénylfluoroformes de formule générale (I) selon la revendication 1 avec des agents diluants et/ou des substances tensioactives.

**Claims**

1. 2-Arylamino-3,5-dinitrobenzotrifluorides of the general formula (I)

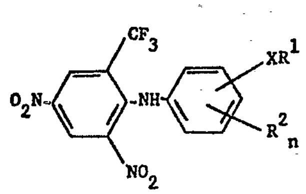

(I)

in which

X represents O, S, SO or $SO_2$,

n represents an integer from 1 to 4,

$R^1$ represents alkyl which is substituted by halogen and/or halogenoalkoxy or phenyl which is optionally substituted by halogen, halogenoalkyl, halogenoalkoxy, halogenoalkylmercapto or halogenoalkylsulphonyl and

$R^2$ represents identical or different radicals from the group comprising hydrogen halogen, cyano, nitro and alkyl which is optionally substituted by halogen and/or halogenoalkoxy,

it being possible for substituents $XR^1$ and $R^2$ in the ortho-position relative to one another, together with the two adjacent C atoms of the phenyl nucleus, to form an optionally halogensubstituted dioxanyl ring; if X represents S, SO or $SO_2$, $R_1$ can represent unsubstituted alkyl, in addition to the radicals indicated above.

2. Process for the preparation of the 2-arylamino-3,5-dinitro-benzotrifluorides of the general formula (I) according to Claim 1, characterized in that

a) an aniline of the general formula (II)

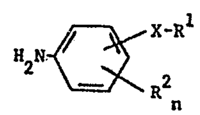

(II)

in which

X, $R^1$, $R^2$ and n have the meaning indicated above, is reacted with 2-chloro-3,5-dinitro-benzotrifluoride of the formula (III)

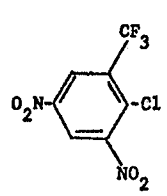

(III)

in the presence of an acid-binding agent and optionally in the presence of a diluent, or